(19) 

**Europäisches Patentamt**
**European Patent Office**
**Office européen des brevets**

(11) **EP 4 653 024 A1**

(12) **EUROPEAN PATENT APPLICATION**

(43) Date of publication:
**26.11.2025 Bulletin 2025/48**

(21) Application number: **24177422.3**

(22) Date of filing: **22.05.2024**

(51) International Patent Classification (IPC):
**A61L 27/46** (2006.01)     **A61L 27/54** (2006.01)
**B33Y 10/00** (2015.01)     **B33Y 80/00** (2015.01)

(52) Cooperative Patent Classification (CPC):
(C-Sets available)
**A61L 27/46; A61L 27/54; B33Y 10/00; B33Y 80/00;**
A61L 2300/414; A61L 2400/06; A61L 2430/02
(Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicants:
  • **Vysoké Uceni Technické V Brne**
    **60200 Brno (CZ)**
  • **Taeknisetur ehf.**
    **112 Reykjavik (IS)**
  • **Genís hf.**
    **580 Siglufjördur (IS)**
  • **Univerzita Karlova, Lekarska fakulta v Plzni**
    **11000 Praha 1 (CZ)**

(72) Inventors:
  • **Vojtova, Lucy**
    **Menin (CZ)**
  • **Örlygsson, Gissur**
    **Mosfellsbaer (IS)**

  • **Vistejnova, Lucie**
    **Kralovice (CZ)**
  • **Kindermann, Marek**
    **Plzen (CZ)**
  • **Chuen, How Ng**
    **Gardabaer (IS)**
  • **Klein, Pavel**
    **Plzen (CZ)**
  • **Michlovska, Lenka**
    **Brno (CZ)**
  • **Hlinakova, Kristyna**
    **Bystrice pod Hostynem (CZ)**
  • **Mencik, Premysl**
    **Vitkov (CZ)**
  • **Brtnikova, Jana**
    **Brno (CZ)**
  • **Lysakova, Klara**
    **Hranice (CZ)**
  • **Kadlecova, Zuzana**
    **Ivancice (CZ)**

(74) Representative: **Harber IP s.r.o.**
  **Dukelskych hrdinu 567/52**
  **17000 Praha 7 (CZ)**

(54) **POLYMER-PHOSPHATE-BASED COMPOSITION FOR 3D PRINTED IMPLANTS**

(57)    The invention provides a composition for low-temperature 3D printing of tissue implants, which contains:
- calcium phosphate powder,
- chitosan particles,
- aqueous solution of block copolymer (D,L-polylactide-co-polyglycolide)-b-poly(ethyleneglycol)-b-(D,L-polylactide-co-polyglycolide) (PLGA-PEG-PLGA).

A kit and a method for preparing the composition are provided, as well as a method for printing tissue implants using the kit.

EP 4 653 024 A1

**(Cont. next page)**

(52) Cooperative Patent Classification (CPC): (Cont.)

C-Sets
**A61L 27/46, C08L 67/04**

**Description**

Field of Art

The present invention relates to a polymer-phosphate-based composition suitable for low-temperature 3D printing of tissue regenerative implants, as well as to a method for the preparation of the composition, and to a method for low-temperature 3D printing of tissue regenerative implants.

Background Art

Since 1958, acrylate cement based on poly(methyl methacrylate) (PMMA) has been used in orthopaedics, most often as a bone glue for the fixation of implants or as a temporary or permanent filling of bone cavities. Bonding and fixation with PMMA is fast and effective with good mechanical stability within minutes. Despite having many advantages such as good handling properties, biointegrity, good biotolerance, suitable biomechanical properties and strength, acrylate bone cements also have several disadvantages - high polymerization temperature, when there is a risk of necrosis of the surrounding tissue, excretion of unreacted toxic monomer or shrinkage. A suitable alternative is injectable degradable calcium phosphate bone cement (CPC), which has wide clinical use as a biocompatible and osteoconductive bone cement with the ability to harden *in vivo* through chemical reactions under physiological conditions. Limitations of calcium phosphate cements used in bone regeneration are their low injectability, low cohesion, disintegration in body fluids (low cohesion), brittleness after curing, slow biodegradability, and low mechanical strength.

Description of the invention

In a first aspect, the present invention provides a kit for preparation of a composition for low-temperature 3D (three-dimensional) printing of tissue implants, wherein the kit contains:

- calcium phosphate powder,
- chitosan particles,
- aqueous solution of block copolymer (D,L-polylactide-co-polyglycolide)-b-poly(ethylene glycol)-b-(D,L-polylactide-co-polyglycolide) (PLGA-PEG-PLGA).

In a second aspect, the present invention provides a composition for low-temperature 3D printing of tissue implants, which contains

- calcium phosphate powder,
- chitosan particles,
- aqueous solution of block copolymer (D,L-polylactide-co-polyglycolide)-b-poly(ethylene glycol)-b-(D,L-polylactide-co-polyglycolide) (PLGA-PEG-PLGA).

In a preferred embodiment of the second aspect, the composition contains

- calcium phosphate powder,
- chitosan particles,
- aqueous solution of block copolymer (D,L-polylactide-co-polyglycolide)-b-poly(ethyleneglycol)-b-(D,L-polylactide-co-polyglycolide) (PLGA-PEG-PLGA) having the concentration within the range of 3 to 30 wt. %,

wherein the weight ratio of the aqueous solution of the block copolymer PLGA-PEG-PLGA to the sum of calcium phosphate powder and chitosan particles is within the range 0.3 to 0.65 (more preferably within the range 0.55 to 0.65), and wherein the amount of chitosan particles is preferably 0.1 to 5 wt. %, relative to the sum of weights of calcium phosphate powder and chitosan particles.

The composition of the invention may be used as an ink or printing paste into 3D printers. The tissue implants which can be printed using the composition of the invention are preferably tissue regeneration implants. An example of the tissue implant is a bone implant.

Low-temperature printing is printing at temperatures below 45 °C, preferably within the range of 0 to 40 °C, more preferably within the range of 10 to 37 °C.

The polymer solution is a thixotropic, fully absorbable aqueous solution of PLGA-PEG-PLGA block copolymer.

The (D,L-polylactide-*co*-polyglycolide)-*b*-poly(ethyleneglycol)-*b*-(D,L-polylactide-*co*-polyglycolide) (PLGA-PEG-PLGA) block copolymer preferably has the general formula (PLAx/PGAy)-PEGz-(PLAx/PGAy), wherein the degree of polymerization z for PEG is in the range from 22 to 35, the degree of polymerization 2x for LA is in the range from 16 to 41 and the degree of polymerization 2y for GA is in the range from 6 to 18.

Particularly preferably, the PLGA-PEG-PLGA copolymer has degrees of polymerization z (PEG) = 34, 2x (LA) = 20.514, and 2y (GA) = 6.038, which correspond to the PLGA/PEG weight ratio equal to 2.5 and the molar ratio of LA/GA equal to 3.0.

LA refers to lactic acid, PLA refers to polylactide, GA refers to glycolic acid, PGA refers to polyglycolide, PEG refers to polyethyleneglycol.

In preferred embodiments, the solvent in the aqueous solution of PLGA-PEG-PLGA is ultrapure water (preferably Type I ultrapure water) or other hypotonic, isotonic and hypertonic aqueous solutions such as physiological solution (0.9 wt. % NaCl aqueous solution), Hartmann's solution, Ringer's solution, Hank's solution, Plasmalyte solution, Ringerfundin, Darrow's solution, or aqueous solutions with defined ionic strength and pH - generally buffers or culture media.

The concentration of PLGA-PEG-PLGA in the aqueous solution can be 3 to 30 wt. %, more preferably 5 to 30 wt. %, even more preferably 10 to 30 wt. %.

The solution of the block copolymer PLGA-PEG-PLGA having the concentration of 20 wt. % in ultrapure water as a solvent is particularly preferred.

Preferably, the aqueous solution further comprises at least one plasticizer and/or at least one emulsifier. The plasticizer can for example be polyethylene glycol with a molecular weight of 200 to 1000 g/mol. The emulsifier may for example be a polysorbate-based emulsifier, preferably selected from polyoxyethylene (20) sorbitan monolaurate (TWEEN 20), polyoxyethylene (40) sorbitan monopalmitate (TWEEN 40), polyoxyethylene (60) sorbitan monostearate (TWEEN 60), polyoxyethylene (65) sorbitan tristearate (TWEEN 65), polyoxyethylene (80) sorbitan monooleate (TWEEN 80). The total amount of plasticizer(s) and/or emulsifier(s) preferably corresponds to a concentration of 2 to 6 wt. %, relative to the weight of the aqueous solution of PLGA-PEG-PLGA. The plasticizers or emulsifiers improve the viscosity and printing properties of the ink.

The plasticizer polyethylene glycol with a molecular weight of 400 g/mol in a concentration of 3-4 wt.%, relative to the weight of the liquid phase (aqueous solution of PLGA-PEG-PLGA) is particularly preferred.

The solid phase (matrix) of the ink is based on bioceramics, namely on calcium phosphates. Calcium phosphate is understood herein as including calcium dihydrogen phosphate monohydrate (MCPM, $Ca(H_2PO_4)_2 \cdot H_2O$, with a molar ratio C/P = 0.5), calcium dihydrogen phosphate anhydrous (MCP, $Ca(H_2PO_4)_2$, C/P = 0.5), calcium hydrogen phosphate dihydrate (DCPD, $CaHPO_4 \cdot 2H_2O$, C/P = 1), calcium hydrogen phosphate anhydrous (DCPA, $CaHPO_4$, C/P = 1), bis(hydrogen phosphate)tetrakis(phosphate) octacalcium pentahydrate (OCP, $Ca_8(HPO_4)_2(PO_4)_4 \cdot 5H_2O$, C/P = 1.33), alpha-tricalcium phosphate (alpha-TCP, $\alpha$-$Ca_3(PO_4)_2$, C/P = 1.5), beta-tricalcium phosphate (beta-TCP, $\beta$-$Ca_3(PO_4)_2$, C/P = 1.5), amorphous calcium phosphate (ACP, $Ca_xH_y(PO_4)_z \cdot nH_2O$, where n = 3-4.5, 15-20% $H_2O$, C/P = 1.2-2.2), calcium-deficient hydroxyapatite (CDHA, $Ca_{10-x}(HPO_4)_x(PO_4)_{6-x}(OH)_{2-x}$, wherein $x$ is between 0 and 2, C/P = 1.50-1.67), hydroxyapatite (HA, $Ca_{10}(PO_4)_6(OH)_2$, C/P = 1.67), fluorapatite (FA, $Ca_{10}(PO_4)_6F_2$, C/P = 1.67), oxyapatite (OA, $Ca_{10}(PO_4)_6O$, C/P = 1.67), tetracalcium bisphosphate (TTCP, $Ca_4(PO_4)_2O$. C/P = 2.0).

The calcium phosphate powder preferably has a particle size in the range of 1 to 200 micrometers, more preferably in the range of 1 to 100 micrometers, even more preferably in the range of 1 to 50 micrometers. It can be preferable that the calcium phosphate powder has a particle size that is less than 100 micrometers, preferably less than 63 micrometers, even more preferably less than 50 micrometers. The maximum particle size is in practice ensured by sieving, and can be determined from SEM photographs or from laser scattering particle size distribution record.

The calcium phosphate powder preferably has a particle size d50 (median particle size) of 1 to 50 micrometers, more preferably 1 to 20 micrometers. The d50 size can be measured by laser scattering (also called laser diffraction) method as a reference method.

The calcium phosphate powder preferably contains or consists of alpha-tricalcium phosphate. In some embodiments, the calcium phosphate powder preferably contains or consists of alpha-tricalcium phosphate and beta-tricalcium phosphate in a ratio of 1:4 (alpha:beta) to 1:6.

To improve the mechanical and osteoconductive properties of the polymer-phosphate ink, the kit and the ink contain a renewable water-soluble biocompatible polysaccharide chitosan. Chitosan has many desirable biological activities such as mucoadhesive, antimicrobial, antitumor, anti-inflammatory, antioxidant, and osteoinductive activities.

Chitosan is partially deacetylated chitin. Chitosan useful in this invention preferably has a weight-average molecular weight within the range from 2 (low molecular) to 400 (high molecular) kg/mol, preferably 100 (low molecular) to 400 (high molecular) kg/mol. This chitosan can have a degree of deacetylation from 35 to 70%, preferably 40 to 60%, more preferably 45 to 55%, even more preferably 50 to 55%. A particle size of less than 20 micrometers, preferably less than 10 micrometers can be advantageous. Any suitable method known in the art for measuring weight average molecular weight can be used. The reference method for measuring weight average molecular weight is preferably SEC/MALS (size exclusion/multi-angle light scattering chromatography). The degree of deacetylation can be determined by NMR analysis or other suitable method, such as spectrophotometry or titration. The reference method for determining the degree of deacetylation is preferably NMR. Particle size can be determined using imaging methods such as SEM or other suitable methods known in the art, including for example laser diffraction or dynamic light scattering. The reference method for determining particle size of chitosan is preferably SEM.

Chitosan can be obtained by deacetylation of chitin from a crustacean. For example, chitosan can be obtained by deacetylation processing of chitin shells from *P. Borealis* shrimp using methods known in the art. Chitosan with a suitable molecular weight is purified for use in the compositions disclosed herein. The chitosan can have a low molecular weight (i.e., a weight-average molecular weight of 2-200 kg/mol, preferably 50-200 kg/mol, more preferably about 100-150 kg/mol, even more preferably about 130 kg/mol). The chitosan can have a high molecular weight (i.e., chitosan having a weight-average molecular weight greater than 200 kg/mol, preferably 250 to 400 kg/mol, more preferably about 250 to 300 kg/mol, even more preferably about 270 kg/mol). The chitosan can have a degree of deacetylation of 35-70%, preferably 40-60%, more preferably 45-55%.

Chitosan can be contained in the composition of the invention in a concentration of 0.1 to 5 wt.% of the solid phase (the solid phase is a sum of calcium phosphate and chitosan). More preferably, the chitosan is contained in the composition of the invention in a concentration of 0.3 to 3 wt.% of the solid phase, even more preferably in a concentration of 1 to 3 wt.% of the solid phase.

In some embodiments, the composition according to the invention further comprises at least one antimicrobial enzyme and/or at least one growth factor. The antimicrobial enzymes may be selected from proteolytic enzymes (e.g., subtilisin, lysostaphin, LL-37, lysine bacteriophages) or polysaccharide degrading enzymes (lysozyme, amylase, dispersin B, alginate lyase). The growth factors may be selected from fibroblast growth factors (FGFs) FGF1 to FGF10 and FGF16 to FGF23, epidermal growth factors (EGFs), vascular endothelial growth factors (VEGFs), and bone morphogenetic proteins (BMPs) from the range of BMP-1 to BMP-8, BMP-10, BMP-11 and BMP-15. Incorporation of specific growth factors can provide specific tissue regenerative activity at the site of introduction, such as for promoting bone regeneration, for wound healing or for other tissue regenerative applications.

Antimicrobial enzyme(s) and/or growth factor(s) may be added either directly to the prepared polymer-phosphate ink or to the liquid phase or solid matrix before their mixing.

Particularly preferably, the antimicrobial enzyme is lysostaphin with a mass of 25 kg/mol. Lysostaphin cleaves cross-linked pentaglycine bridges in the bacterial cell wall peptidoglycan, and has antibacterial efficacy, especially against epidemiologically significant sequence types (ST) of *Staphylococcus aureus* commonly found in chronic infections.

For bone healing applications, the growth factor can preferably be fibroblast growth factor 2 (FGF2), more preferably in the form of a recombinant human protein produced by *E. coli*.

In some embodiments, antimicrobial enzyme(s) and/or growth factor(s) are in the form of core-shell particles, in which the antimicrobial enzyme(s) and/or growth factor(s) is/are encapsulated into a lipidic or polymeric carrier. Encapsulation protects the antimicrobial enzyme(s) and/or growth factor(s), both chemically and physically, from changes in pH, temperature, or mechanical stresses that may be caused by the surrounding implant cement matrix which is formed by curing the composition. Suitable carrier systems include lipid nanoparticles (called liposomes) and polymer nanoparticles (called polymersomes), primarily due to their ability to capture both hydrophobic and hydrophilic substances, including proteins and enzymes. Due to the needle-like crystalline structure of the cured cement (implant), liposomes may not provide sufficient mechanical support due to their easy deformability. This property, although advantageous in other applications, may not yield the desired results in the case of bone cement. Therefore, by exploiting the properties of materials already present in cement, core-shell polymersomes based on a triblock thermosensitive copolymer poly(lactic-*co*-glycolic acid)-b-poly(ethylene glycol)-b-poly(lactic-*co*-glycolic acid) (PLGA-PEG-PLGA) which can be prepared by the double emulsion (water-in-oil-in-water) method are especially preferred. Another advantage of these particles is their ability to control the rate of degradation and drug release according to the molecular weight of the triblock copolymer and the ratio of PEG to PLGA. Efficiently prepared core-shell particles are biodegradable, biocompatible, and have low toxicity, while effectively protecting the active ingredient and maintaining their submicron diameter.

In a third aspect of the invention, a method of preparing the composition for low-temperature 3D printing of tissue implants is provided, comprising the steps of:

- providing an aqueous solution of block copolymer (D,L-polylactide-*co*-polyglycolide)-*b*-poly(ethylene glycol)-*b*-(D,L-polylactide-*co*-polyglycolide) (PLGA-PEG-PLGA), preferably having the concentration within the range of 3 to 30 wt. %;
- dissolving chitosan in the aqueous solution of the block copolymer PLGA-PEG-PLGA;
- providing calcium phosphate powder;
- combining the aqueous solution of the block copolymer PLGA-PEG-PLGA and chitosan with calcium phosphate powder.

The aqueous solution of PLGA-PEG-PLGA may in some embodiments contain one or more additives selected from plasticizers, emulsifiers, antimicrobial enzymes, and growth factors.

The step of dissolving chitosan in the aqueous solution of the block copolymer PLGA-PEG-PLGA is preferably carried out by stirring at 1000-2000 rpm, preferably using a planetary centrifugal mixer.

The step of combining the aqueous solution of the block copolymer PLGA-PEG-PLGA and chitosan with calcium phosphate powder is preferably carried out by mixing at 1000-3000 rpm, preferably using a planetary centrifugal mixer.

In a fourth aspect of the invention, a method of low-temperature 3D printing of tissue implants is provided, comprising the steps of:

- providing an aqueous solution of block copolymer (D,L-polylactide-*co*-polyglycolide)-*b*-poly(ethyleneglycol)-*b*-(D,L-polylactide-*co*-polyglycolide) (PLGA-PEG-PLGA), preferably having the concentration within the range of 3 to 30 wt. %;
- dissolving chitosan in the aqueous solution of the block copolymer PLGA-PEG-PLGA;
- providing calcium phosphate powder;
- combining the aqueous solution of the block copolymer PLGA-PEG-PLGA and chitosan with calcium phosphate powder to produce a printable composition;
- printing tissue implant(s) using the printable composition at the temperature within the range from 0 to 45 °C, preferably using discontinuous extrusion method;
- curing the printed object(s) in aqueous liquid or in air with at least 95 % of humidity.

The 3D printable composition has a solidification time, during which it can be printed, from 20 to 60 minutes, depending on the composition ratio.

The printed implants are cured in a humid environment or in aqueous liquids. Aqueous liquids may include physiological solution, Hartmann's solution, Ringer's solution, Hank's solution, Plasmalyte solution, Darrow's solution, Ringerfundin, biological fluids such as plasma or blood, water-based synthetic biological fluids, buffers such as PBS, and culture media.

The present invention further provides tissue implants obtainable by the above-described method. The implants may, in some embodiments, have a grid structure selected from orthogonal, gyroid, and honeycomb patterns. The infill density may range, for example, from 50 to 100% for orthogonal and gyroid patterns and by setting the number of honeycombs (fine, medium, coarse) in the structure. By choosing the grid structure and/or its further parameters, the concentration of individual components, and the method of curing, suitable mechanical properties can be set depending on the potential use of the printed implant.

Optionally, the implants may be coated by at least one antimicrobial enzyme and/or at least one growth factor which are described herein above. The coating may be performed by immersing the printed and cured implant in a solution of the protein for at least one hour, more preferably for at least 12 hours. The solvent may be ultrapure water (preferably Type I ultrapure water) or other hypotonic, isotonic, and hypertonic aqueous solutions such as physiological solution (0.9 wt. % NaCl aqueous solution), Hartmann's solution, Ringer's solution, Plasmalyte solution, Ringerfundin, Darrow's solution, or aqueous solutions with defined ionic strength and pH - generally buffers.

Improvement of the properties of the polymer-phosphate composition relies on the thermosensitive, biodegradable, thixotropic copolymer based on polylactic acid, polyglycolic acid and polyethylene glycol (PLGA-PEG-PLGA), which undergoes gelation under physiological conditions, and on the addition of biocompatible, degradable polysaccharide chitosan which improves mechanical properties and can promote bone healing and osteointegration of 3D printed implants. In the treatment of a bone fracture or other tissue, as well as during fixation of the implant, contamination of the surgical site with pathogenic bacteria or extensive inflammation, which begins immediately after injury and lasts for several days, may also occur. In the area of the fracture, there is damage to blood vessels, bleeding, and formation of hematomas caused by necrosis of osteocytes due to hypoxia and subsequent thrombosis. For bone cements, these complications are solved by the addition of antibiotics to the bone cements. As a result of the overuse of antibiotics and the increasing resistance of a number of bacterial strains, there is pressure to replace antibiotic preparations with other types of suitable antimicrobial therapeutics, e.g. antibacterial enzymes, or healing growth factors.

Examples

Chemicals

All chemicals used for chemical reactions/instrumental analyses were reagent-grade chemicals, and all (apart from the below-listed exceptions) were purchased from Sigma-Aldrich.

Sodium hydroxide and hydrochloric acid used in the production processes were food-grade chemicals purchased from Tandur (Iceland).

Alpha-tricalcium phosphate with a declared particle size of d50 $\leq$ 15 $\mu$m was purchased from Innotere Biomaterials (Germany). The particle size distribution was verified by Laser Scattering Particle Size Distribution Analyzer LA-950 Horiba. TCP powder was dispersed in isopropanol, and after 30 minutes of ultrasonication particle size distribution was measured. The results confirmed a bimodal distribution character with particle sizes from 1 to 174 μm. However, particles above 100 μm were not desirable for the preparation of polymer-phosphate printing compositions (inks) under our laboratory conditions due to the possible clogging of the thin nozzle during the printing process. Therefore, the TCP powder was sieved through a 63 μm sieve. After that, the particle size distribution analysis was again performed, which showed that the particle size ranged from 1 to 23 μm. The average particle size corresponded to 6.45 μm, median particle size 6.12, diameter on cumulative percentage - d10(%) - 2.85 and d90(%) - 11.39.

Chitosan (partially deacetylated chitin, PDC; ChitoBiomer™ Grade) with a 50% degree of deacetylation and two molecular weights (approx. 100 and 300 kg/mol) derived from P. Borealis shrimp shells is commercially available from Genis (Iceland). The properties of chitosan were verified by measurements:

## Morphology of chitosan by scanning electron microscopy (SEM)

The morphology of PDC particulates was conducted by Zeiss Gemini SEM. Before analysis, the samples were coated with a gold layer for 4 minutes using Edward's sputter coater, S105B. The SEM image showed that the chitosan material is in the form of spherical microparticles with sizes ranging from 1-10 μm. The fine particulate facilitates easy mixing/dissolution with other components, e.g. calcium phosphates, to maximize the homogeneity of the mixture.

## Chemical composition of chitosan by Fourier-transformed infra-red spectroscopy

The attenuated total reflection-Fourier transform infrared (ATR-FTIR) spectra were measured in transmission mode between 400-4000 cm$^{-1}$ with Nicolet iZ10 MX / microscopic IR, Nicolet iN10 MX (Thermo Scientific) spectrophotometer with 64 measurement cycles. The band at 1648 cm$^{-1}$ corresponded to amide I vibration and a well-defined band at 1556 cm$^{-1}$ to amide II or N-H bending.

This amide II band is an important indicating band for N-acetyl-D-glucosamine. These findings were in good agreement with previous report results [DUARTE, M.L, FERREIRA, M.C, MARVAO, M.R and ROCHA, J. An optimised method to determine the degree of acetylation of chitin and chitosan by FTIR spectroscopy. International Journal of Biological Macromolecules [online]. 2002. Vol. 31, no. 1-3p. 1-8. DOI 10.1016/S0141-8130(02)00039-9.]. Other important indicators of PDC include the band 2913 cm$^{-1}$ (C-H stretch), the band 1379 cm$^{-1}$ for $CH_3$ in the amide group, and the band 1110 cm$^{-1}$ for the vibration of the polysaccharide backbone [ALTIOK, D., ALTIOK, E. and TIHMINLIOGLU, F. Physical, antibacterial and antioxidant properties of chitosan films incorporated with thyme oil for potential wound healing applications. Journal of Materials Science: Materials in Medicine [online]. 2010. Vol. 21, no. 7p. 2227-2236. DOI 10.1007/s10856-010-4065-x]. The β-(1→4)-glycosidic bond is visible at 894 cm$^{-1}$.

## Degree of deacetylation of chitin (chitosan) by nuclear magnetic resonance analysis

Proton $^1$H NMR spectroscopy analysis of the chitin degree of deacetylation was done according to the method of [HIRAI, A., ODANI, H. and NAKAJIMA, A. Determination of degree of deacetylation of chitosan by 1H NMR spectroscopy. Polymer Bulletin [online]. 1991. Vol. 26, no. 1p. 87-94. DOI 10.1007/BF00299352; LAVERTU, M., XIA, Z., SERREQI, A.N., BERRADA, M., RODRIGUES, A., WANG, D., BUSCHMANN, M.D. and GUPTA, A. A validated 1H NMR method for the determination of the degree of deacetylation of chitosan. Journal of Pharmaceutical and Biomedical Analysis [online]. 2003. Vol. 32, no. 6p. 1149-1158. DOI 10.1016/S0731-7085(03)00155-9]. Briefly, the $^1$H NMR spectra of 1% PDC in a mixture of 0.5% deuterated chloroform in $D_2O$ were recorded on a Bruker spectrometer at 400 MHz and 67 °C. The degree of acetylation was calculated by comparing the integrated area under the peaks associated with H2-H6 and that with the acetyl group, as described in the article. The acetyl group peak is at δ~2.4 ppm. The resonance of the H3-H6 ring proton of glucosamine and H2-H6 is in the middle of the spectrum (δ~3.9-4.2). H-1D of internal deacetylation units resonates at 5.2 ppm. The degree of deacetylation (DD) can be calculated by using Equation 1:

$$DD = [1-((1/3Hac)/(1/6H-6))] \times 100\ (\%) \qquad (Eq.\ 1)$$

The DD obtained from this equation were 51.6 % and 51.4 %, for low and high MW PDC, respectively.

## Molecular weight analysis by size exclusion chromatography (SEC)

The analysis of weight-average molecular weight ($M_w$) of the two PDCs was conducted by SEC/MALS (Size exclusion/multi-angle light scattering chromatography). The detectors were HELEOS-II and the RI, Optilab T-rEX, both from Wyatt Technology Corporation, and the chromatographic data were evaluated using Astra software. A combination of PL aquagel-OH Mixed M 8 μm/PL aquagel-OH Mixed H 8 μm columns with the mobile phase of a mixture of acetate buffer (i.e., 0.5M acetic acid + 0.2M sodium acetate) was applied. The flow rate of the mobile phase was 0.8 ml/min with an injection volume of 100 μl. The dn/dc value for chitosan in acetate buffer was 0.181 ml/g. The analytical dextran was used as the standard having a molecular weight of 25 000 g/mol, and all samples were measured five times.

| Sample | $M_w$ (kg/mol) | $M_n$ (kg/mol) | PDI - polydispersity index $M_n/M_w$ |
|---|---|---|---|
| Low MW chitosan | 133 | 59 | 2.26 |
| High MW chitosan | 274 | 177 | 1.55 |

The printable composition is called "ink" in the examples, and has the form of a paste.

**3D printing procedure:**

During 3D printing, the material (ink) is located in a reservoir with a volume of 3 to 10 ml. It is pushed out of this reservoir by a piston, on which pressure is exerted mechanically by a lead screw, or pneumatically. The material is extruded through a nozzle with a 250, or 410 μm diameter. The pressure required to extrude the material depends on the nozzle diameter, the viscosity of the printed paste, and the printing speed. It varies between 80 and 300 kPa. The optimal height of the print layer is between 0.5 and 0.8 times the diameter of the extrusion nozzle. Optimum print speeds range from 5 to 15 mm/s. The implants can be printed on commercial 3D bio-printing printers such as Cellink's BioX, which uses air pressure for 3D printing. Other variants are custom modified FDM 3D printers or 3D printers for extruding paste and cement materials, such as the Zmorph VX. For each printer, a print model must be prepared using slicing programs, or slicers for short. These will prepare the control code for the printer. Most printers have a slicer that is ready for printing liquid materials from the manufacturer, or these programs have to be manually modified for printing liquid materials. A slicer prepared by the manufacturer is, for example, HearthWare from Cellink, or Voxelizer for the Zmorph VX printer. Third-party open-source slicing programs, such as Prusa Slicer, can also be advantageously used.

**Example 1: Polymer-phosphate ink based on calcium phosphate, low molecular weight chitosan and PLGA-PEG-PLGA**

The polymer-phosphate ink was prepared by combining the liquid (L) and solid (P) (or powder) phases in the ratio L/P = 0.62. The liquid phase consists of an aqueous solution of thermosensitive triblock copolymer based on degradable polylactic acid (PLA), polyglycolic acid (PGA), and soluble polyethylene glycol) (PLGA-PEG-PLGA) at a concentration of 20 wt.%. Type I ultrapure water was used as the solvent. The triblock copolymer with a theoretical molecular weight of 5250 g/mol was prepared at 130 °C by ring-opening polymerisation under an inert atmosphere. The solid phase consists of commercially available alpha-tricalcium phosphate (TCP) with a particle size <63 μm and low molecular weight chitosan (LMWCh) with a concentration of 1.8 wt.%, relative to the weight of the solid phase.

Polymer-phosphate ink was prepared by homogenization using a planetary centrifugal mixer, wherein the chitosan was first mixed into the copolymer solution (10 min, 1500 rpm). Then the remaining TCP powder was added (2 min, 2000 rpm). The polymer-phosphate ink containing chitosan and TCP as the solid phase and the PLGA-PEG-PLGA solution as the liquid phase was used immediately to print cylinders of 6×12 mm (diameter × high) with an orthogonal pattern and an infill density of 50%, using a nozzle with a diameter of 410 μm. The printed cylinders were cured in a humid environment of 95-100 % at 37 °C for 3 days.

**Example 2: Polymer-phosphate ink with emulsifier Tween 80**

The polymer-phosphate ink was prepared as described in Example 1, but 4 wt.% (relative to the weight of the liquid phase) of the emulsifier Tween 80 was added to the liquid phase, after the addition of chitosan. The mixture was then stirred (1 min, 1500 rpm) before the addition of the remaining TCP powder. The prepared polymer-phosphate ink was used immediately to print cylinders of 6×12 mm (diameter × high) with an orthogonal pattern and infill density of 50%, using a nozzle with a diameter of 410 μm. The printed cylinders were cured in a humid environment at 37 °C for 3 days.

**Example 3: Polymer-phosphate ink with plasticizer PEG 400**

The polymer-phosphate ink was prepared as described in Example 1, but 4 wt.% (relative to the weight of the liquid phase) of the plasticizer polyethylene glycol with a molecular weight of 400 g/mol (PEG 400) was added to the liquid phase, after addition of chitosan. The mixture was then stirred (1 min, 1500 rpm) before the addition of the remaining TCP solid phase.

The prepared polymer-phosphate ink was used immediately to print cylinders of 6×12 mm (diameter × high) with an orthogonal pattern and infill density of 50% using a nozzle with a diameter of 410 μm. The printed cylinders were cured in a humid environment at 37 °C for 3 days.

**Example 4: Polymer-phosphate ink with plasticizer PEG 600**

The polymer-phosphate ink was prepared as described in Example 1, but 4 wt.% (relative to the weight of the liquid phase) of the plasticizer polyethylene glycol with a molecular weight of 600 g/mol (PEG 600) was added to the liquid phase, after addition of chitosan. The mixture was then stirred (1 min, 1500 rpm) before the addition of the remaining TCP powder.

The prepared polymer-phosphate ink was used immediately to print cylinders of 6×12 mm (diameter × high) with an orthogonal pattern and an infill density of 50%, using a nozzle with a diameter of 410 μm. The printed cylinders were cured in a humid environment at 37 °C for 3 days.

In Examples 1-4, the effect of the plasticizer/emulsifier and its type on the mechanical properties in compression was investigated.

The mechanical properties in compression were measured using a universal testing machine (Z010 TE Allround-Line, Zwick/Roell) with a load cell of 1 kN and a strain rate of 1 mm/min. This method was used to evaluate the compressive strength of the printed specimens (MPa), which was calculated from the ratio of the standard force F (N) to the area A of each specimen ($mm^2$). The compressive strength results depending on the used plasticiser are shown in **Table 1.**

**Table 1.** Compressive strength of cured printed cylinders of 6×12 mm Example 1-4 with orthogonal pattern and infill density of 50%.

| Example | Plasticizer / Emulsifier | Compressive strength (MPa) | | |
|---|---|---|---|---|
| | | average | median | deviation |
| Example 1 | - | 6.76 | 6.30 | 0.99 |
| Example 2 | TWEEN 80 | 6.05 | 6.18 | 0.60 |
| Example 3 | PEG 400 | 6.49 | 6.38 | 0.54 |
| Example 4 | PEG 600 | 7.78 | 7.59 | 0.67 |

The samples with PEG 600 plasticizer (Example 4) at 4 wt.% of the liquid phase showed the highest compressive strengths with statistical significance compared to the other groups of samples, with the median strength increasing by 20% from 6.30 to 7.59 MPa compared to Example 1 samples prepared without plasticizer. The specimens with TWEEN 80 emulsifier (Example 2) and PEG 400 (Example 3) had comparable mechanical properties to the Example 1 samples.

In Examples 1-4, the effect of the addition and type of plasticizer on the conversion of the original α-TCP to calcium-deficient hydroxyapatite after curing was investigated using X-ray powder diffraction (Rigaku SmartLab 3kW, Rigaku, Japan). The diffraction patterns were measured from 5° to 50°. The conversion was calculated from the intensities of the selected peaks and is shown in Table 2.

**Table 2.** Percentage conversion of α-TCP to CDHA for printed samples without plasticizer/emulsifier (Example 1) and with different types of plasticizers/emulsifiers (Example 2-4).

| Example | Plasticizer / Emulsifier | CDHA conversion (%) |
|---|---|---|
| Example 1 | - | 78.6 |
| Example 2 | TWEEN 80 | 83.6 |
| Example 3 | PEG 400 | 81.7 |
| Example 4 | PEG 600 | 75.1 |

X-ray powder diffraction confirmed the opposite trend to the mechanical tests, where the samples for Example 2 (with TWEEN 80 addition) and Example 3 (with PEG 400 addition) showed slightly higher conversion of TCP to calcium-deficient hydroxyapatite than the control samples. Although the samples from Example 4 (with the addition of PEG 600) had a higher compressive strength, the conversion was lower than in the other specimens. It is clear from the values that the addition and type of plasticizer/emulsifier affects the resulting conversion, which is lowest when using PEG 600 plasticizer (75.1%), followed by no plasticizer (78.6%) and PEG 400 (81.7%). The best conversion was achieved by the printed samples with TWEEN 80 emulsifier (83.6%).

Conclusion: The compressive strength results of the printed samples were not statistically significantly different. The use of plasticizers/emulsifier does not have a significant adverse effect on the mechanical properties. The use of PEG 400 as a plasticizer with a concentration of 4 wt.% of the liquid phase was found to be the most suitable, where the median strength increased from 6.30 to 6.38 MPa and the conversion increased by 4% compared to Example 1 without plasticiser.

**Example 5: Polymer-phosphate ink, 1.0 wt.% LMW chitosan**

Polymer-phosphate ink was prepared by combining the liquid (L) and solid (P) phases in the ratio L/P = 0.62. The liquid phase contains an aqueous solution of thermosensitive triblock copolymer based on degradable polylactic acid (PLA), polyglycolic acid (PGA), and soluble polyethylene glycol) (PLGA-PEG-PLGA) at a concentration of 20 wt.%. Type I ultrapure water was used as the solvent. The liquid phase further contains 4 wt.% (relative to the weight of the liquid phase) of the plasticizer poly(ethylene) glycol with a molecular weight of 400 g/mol (PEG 400). The solid phase contains commercially available alpha-tricalcium phosphate (TCP) with a particle size <63 $\mu$m and low molecular weight chitosan (LMWCh) with a concentration of 1.0 wt.%, relative to the solid phase (sum of the weights of TCP and chitosan).

Polymer-phosphate ink was prepared by homogenization using a planetary centrifugal mixer, wherein the chitosan was first mixed into the copolymer solution (10 min, 1500 rpm), then the plasticizer was stirred into the mixture (1 min, 1500 rpm). Then the TCP powder was added (2 min, 2000 rpm). The polymer-phosphate ink was used immediately to print cylinders of 6×12 mm (diameter × high) with an orthogonal pattern and an infill density of 50%, using a nozzle with a diameter of 410 $\mu$m. The printed cylinders were cured in a humid environment at 37 °C for 3 days.

**Example 6: Polymer-phosphate ink, 1.5 wt.% LMW chitosan**

Polymer-phosphate ink was prepared as described in Example 5, but 1.5 wt.% LMW chitosan, relative to the solid phase, was used.

The prepared polymer-phosphate ink was used immediately to print cylinders of 6×12 mm (diameter × high) with an orthogonal pattern and a density of infill 50%, using a nozzle with a diameter of 410 $\mu$m. The printed cylinders were cured in a humid environment at 37 °C for 3 days.

**Example 7: Polymer-phosphate ink, 2 wt.% LMW chitosan**

Polymer-phosphate ink was prepared as described in Example 5, but 2.0 wt.% LMW chitosan, relative to the solid phase, was used.

The prepared polymer-phosphate ink was used immediately to print cylinders of 6×12 mm (diameter × high) with an orthogonal pattern and a density of infill 50%, using a nozzle with a diameter of 410 $\mu$m. The printed cylinders were cured in a humid environment at 37 °C for 3 days.

**Example 8: Polymer-phosphate ink, 2.5 wt.% LMW chitosan**

Polymer-phosphate ink was prepared as described in Example 5, but 2.5 wt.% LMW chitosan, relative to the solid phase, was used.

The prepared polymer-phosphate ink was used immediately to print cylinders of 6×12 mm (diameter × high) with an orthogonal pattern and a density of infill 50%, using a nozzle with a diameter of 410 $\mu$m. The printed cylinders were cured in a humid environment at 37 °C for 3 days.

**Example 9: Polymer-phosphate ink, 1 wt.% HMW chitosan**

Polymer-phosphate ink was prepared as described in Example 5, but 1.0 wt.% HMW (high-molecular weight) chitosan, relative to the solid phase, was used.

The prepared polymer-phosphate ink was used immediately to print cylinders of 6×12 mm (diameter × high) with an orthogonal pattern and a density of infill 50%, using a nozzle with a diameter of 410 $\mu$m. The printed cylinders were cured in a humid environment at 37 °C for 3 days.

**Example 10: Polymer-phosphate ink, 1.5 wt.% HMW chitosan**

Polymer-phosphate ink was prepared as described in Example 5, but 1.5 wt.% HMW chitosan, relative to the solid phase, was used.

The prepared polymer-phosphate ink was used immediately to print cylinders 6×12 mm (diameter × high) with an orthogonal pattern and a density of infill 50%, using a nozzle with a diameter of 410 μm. The printed cylinders were cured in a humid environment at 37 °C for 3 days.

**Example 11: Polymer-phosphate ink, 1.8 wt.% HMW chitosan**

Polymer-phosphate ink was prepared as described in Example 5, but 1.8 wt.% HMW chitosan, relative to solid phase, was used.

The prepared polymer-phosphate ink was used immediately to print cylinders of 6×12 mm (diameter × high) with an orthogonal pattern and a density of infill 50%, using a nozzle with a diameter of 410 μm. The printed rollers were cured in a humid environment at 37 °C for 3 days.

In Example 3 and Examples 5-11, the effect of the type of chitosan (low molecular weight or high molecular weight) and its concentration on the consistency and mechanical properties of the 3D printed samples was investigated using a nozzle 410 μm in the form of 6×12 mm (diameter × high) cylinders with infill density of 50% and an orthogonal pattern. The mechanical properties were measured and monitored using the same procedure as in the previous examples, and the results are shown in Table 3.

**Table 3.** Compressive strength of cured printed 6×12 mm cylinders with orthogonal pattern and 50% infill density, using low molecular weight and high molecular weight chitosan at different concentrations (Examples 3, 5-11).

| Example | Chitosan | Chitosan concentration | Compressive strength (MPa) | | |
|---------|----------|------------------------|---------|--------|-----------|
| | | (wt.% of solid phase) | average | median | deviation |
| Example 5 | LMWCh | 1.0 | 5.89 | 6.35 | 1.11 |
| Example 6 | LMWCh | 1.5 | 6.73 | 6.60 | 1.11 |
| Example 3 | LMWCh | 1.8 | 6.79 | 6.88 | 0.83 |
| Example 7 | LMWCh | 2.0 | 5.60 | 5.50 | 2.37 |
| Example 8 | LMWCh | 2.5 | 7.46 | 7.11 | 1.13 |
| Example 9 | HMWCh | 1.0 | 4.94 | 5.26 | 0.89 |
| Example 10 | HMWCh | 1.5 | 9.12 | 8.94 | 1.50 |
| Example 11 | HMWCh | 1.8 | 9.53 | 9.56 | 0.87 |

In terms of the consistency of the polymer-phosphate ink, the ink in Example 5 was rather thin and did not provide quite the desired printing properties compared to the other Examples with higher concentrations of chitosan. A slightly increasing trend in compressive strength can be observed for the low molecular weight chitosan (Examples 5-8), where the median compressive strength increased from 6.35 MPa (Example 5, LMWCh concentration 1.0 wt.%) to 7.11 MPa (Example 8, LMWCh concentration 2.5 wt.%). According to the statistical analysis, there is a significant difference only between Example 5 and Example 8, when low molecular weight chitosan was used. The small increments in strength were caused by the amount of chitosan being calculated as part of the solid phase; the higher the amount of chitosan, the lower the amount of α-TCP in the sample.

The increment in strength between Example 9, Example 10, and Example 11 with increasing concentration of high molecular weight chitosan is much more significant, with the median strength increasing from 5.26 MPa for 1 wt.% HMWCh concentration of the solid phase used (Example 9) by 70% for Example 10 (1.5 wt.% HMWCh of the solid phase used) and 82% for Example 11 (1.8 wt.% HMWCh of the solid phase used).

Conclusions: The results show that increasing the chitosan concentration positively affects the mechanical properties in compression. The mechanical properties at higher concentrations also depend on the type of chitosan, where the compressive strength increased by 37% on average when using high molecular weight chitosan at concentrations of 1.5 and 1.8 wt.%.

**Example 12: Polymer-phosphate ink with LMW chitosan, orthogonal pattern**

Polymer-phosphate ink was prepared by combining the liquid (L) and solid (P) phases in the ratio L/P = 0.62. The liquid phase contains an aqueous solution of thermosensitive triblock copolymer based on degradable polylactic acid (PLA), polyglycolic acid (PGA), and soluble polyethylene glycol) (PLGA-PEG-PLGA) at a concentration of 20 wt.%. Type I ultrapure water was used as the solvent. The liquid phase further contains 4 wt.% (relative to the weight of the liquid phase) of the plasticizer poly(ethylene) glycol with a molecular weight of 400 g/mol (PEG 400). The solid phase contains commercially available alpha-tricalcium phosphate (TCP) with a particle size <63 $\mu$m and low molecular weight chitosan (LMWCh) with a concentration of 1.8 wt.%, relative to the solid phase..

Polymer-phosphate ink was prepared by homogenization using a planetary centrifugal mixer, wherein the chitosan was first mixed into the copolymer solution (10 min, 1500 rpm), then the plasticizer was stirred into the mixture (1 min, 1500 rpm). Then the TCP powder was added (2 min, 2000 rpm).

The prepared polymer-phosphate ink was used to print 8 cuboids of 6×6× height 12 mm with an orthogonal pattern and an infill density of 50%, using a nozzle with a diameter of 410 $\mu$m. The printed cuboids were cured in a humid environment at 37 °C for 3 days.

**Example 13: Polymer-phosphate ink with LMW chitosan, gyroid pattern**

Polymer phosphate ink was prepared as described in example 12.

The prepared polymer-phosphate ink was used to print 8 cuboids of 6×6×12 mm in height with a gyroid pattern and an infill density of 50%, using a nozzle with a diameter of 410 $\mu$m. The printed cuboids were cured in a humid environment at 37 °C for 3 days.

**Example 14: Polymer-phosphate ink with LMW chitosan, honeycomb pattern**

Polymer phosphate ink was prepared as described in Example 12.

The prepared polymer-phosphate ink was used to print 8 cuboids of 6×6×12 mm in height with a honeycomb pattern with a ratio of 1.0 and an infill density of 50%, using a nozzle with a diameter of 410 $\mu$m. The printed cuboids were cured in a humid environment at 37 °C for 3 days.

**Example 15: Polymer-phosphate ink with LMW chitosan, honeycomb pattern**

Polymer phosphate ink was prepared as described in Example 12.

The prepared polymer-phosphate ink was used to print 8 cuboids with a size of 6×6×12 mm in height with a honeycomb pattern with a ratio of 1.5 and an infill density of 50%, using a nozzle with a diameter of 410 $\mu$m. The printed cuboids were cured in a humid environment at 37 °C for 3 days.

**Example 16: Polymer-phosphate ink with LMW chitosan, honeycomb pattern**

Polymer phosphate ink was prepared as described in Example 12.

The prepared polymer-phosphate ink was used to print 8 cuboids with a size of 6×6×12 mm in height with a honeycomb pattern with a ratio of 2.0 and an infill density of 50%, using a nozzle with a diameter of 410 $\mu$m nozzle. The printed cuboids were cured in a humid environment at 37 °C for 3 days.

For **Examples 12-16,** the effect of the pattern of the 3D printed samples on the mechanical properties in compression was investigated. The samples were printed using a 410 $\mu$m nozzle in the form of 6×6×12 mm cuboids with an infill density of 50%. The mechanical properties were measured and monitored using the same procedure as in the previous examples and the results are shown in Table 4.

**Table 4.** Compressive strength of cured printed cuboids (6×6×12 mm) with an infill density of 50%, using low molecular weight chitosan with different patterns (Example 12-16).

| Example | Pattern | Compressive strength (MPa) | | |
|---|---|---|---|---|
| | | average | median | deviation |
| Example 12 | orthogonal | 8.35 | 8.53 | 1.81 |
| Example 13 | gyroid | 7.16 | 7.44 | 1.64 |
| Example 14 | honeycomb 1.0 | 16.23 | 16.24 | 3.19 |
| Example 15 | honeycomb 1.5 | 14.93 | 15.22 | 2.04 |
| Example 16 | honeycomb 2.0 | 8.60 | 8.32 | 1.10 |

From Table 4, the choice of the internal structure of the printed specimens while maintaining the infill density can control the resulting mechanical properties of the implants in terms of compressive strength. For the examples shown, the median compressive strength ranged from 8.32 MPa for Example 16 (honeycomb 2.0). There was no statistical significance between Example 12, Example 13, and Example 16 for the measured median compressive strengths. The median compressive strength increased significantly for Example 15 (honeycomb 1.5, 15.22 MPa) and Example 14 (honeycomb 1.0, 16.24 MPa).

**Example 17: Polymer-phosphate ink with LMW chitosan and plasticizer**

Polymer-phosphate ink was prepared by combining the liquid (L) and powder (P) (or solid) phases in the ratio L/P = 0.62. The liquid phase contains an aqueous solution of thermosensitive triblock copolymer based on degradable polylactic acid (PLA), polyglycolic acid (PGA), and soluble polyethylene glycol) (PLGA-PEG-PLGA) at a concentration of 20 wt.%. Type I ultrapure water was used as the solvent. The liquid phase further contains 3 wt.% (relative to the weight of the liquid phase) of the plasticizer poly(ethylene) glycol with a molecular weight of 400 g/mol (PEG 400). The solid phase contains commercially available alpha-tricalcium phosphate (TCP) with a particle size <63 $\mu$m and low molecular weight chitosan (LMWCh) with a concentration of 1.8 wt.%, relative to the solid phase.

Polymer-phosphate ink was prepared by homogenization using a planetary centrifugal mixer, wherein the chitosan was first mixed into the copolymer solution (10 min, 1500 rpm), then the plasticizer was stirred into the mixture (30 seconds, 1500 rpm). Then the TCP powder was added (2 min, 2000 rpm).

The prepared polymer-phosphate ink was used immediately to print the discs with diameter $\times$ height 6$\times$0.35 mm (for release monitoring), 6$\times$2 mm (for *in vitro* testing), and 3.5$\times$1 mm (for *in vivo* testing) with an orthogonal pattern and an infill density of 50%, using a nozzle with a diameter of 250 $\mu$m. The printed discs were cured in a humid environment at 37 °C for 3 days.

**Example 18: Polymer-phosphate ink with HMW chitosan and plasticizer**

The polymer-phosphate ink was prepared as described in Example 17, but 1.8 wt.% HMW chitosan, relative to the solid phase, was used. The prepared polymer-phosphate ink was used immediately to print the discs with diameter $\times$ height 6$\times$0.35 mm (for release monitoring), 6$\times$2 mm (for *in vitro* testing), and 3.5$\times$1 mm (for *in vivo* testing) with an orthogonal pattern and an infill density of 50%, using a nozzle with a diameter of 250 $\mu$m. The printed discs were cured in a humid environment at 37 °C for 3 days.

**Example 19: Polymer-phosphate ink with LMW chitosan loaded with FGF2**

Polymer-phosphate ink was prepared as in Example 17. To the prepared ink, FGF2 protein solution (commercially available) was added in the amount to reach the final amount of 1 $\mu$g FGF2 per printed sample. An adequate amount of TCP was added to the solid phase to keep the liquid-to-solid ratio of L/P = 0.62, thus compensating for the addition of the FGF2 protein solution.

Printing procedure of the samples was as in Example 17.

**Example 20: Polymer-phosphate ink with LMW chitosan loaded with lysostaphin**

Polymer-phosphate ink was prepared as in Example 17. To the prepared ink, lysostaphin protein (enzyme) solution (commercially available) was added in the amount to reach the final amount of 10 $\mu$g lysostaphin per printed sample. An adequate amount of TCP was added to the solid phase to keep the liquid-to-solid ratio of L/P = 0.62, thus compensating for the addition of the lysostaphin solution.

Printing of the samples was as in Example 17.

**Example 21: Encapsulation procedure**

The preparation of core-shell nanoparticles by the double emulsion water-in-oil-in-water (W/O/W) method involved the dissolution of the PLGA-PEG-PLGA copolymer in acetone to form a 10% w/v solution. In the following step, PBS (150 mM) was added dropwise to the polymer solution. To prepare empty particles, PBS alone was added to prepare protein/enzyme-loaded particles and a protein solution in PBS was added to the solution until an emulsion was formed. Subsequently, the emulsion was sonicated using a bath sonicator (Czech Republic) at 4 °C for 10 min, after which a 1 % w/v solution of polyvinyl alcohol (PVA) in ultrapure Type I water was added as an emulsion protectant, and the mixture was gently mixed at 250 rpm at 12 °C for 30 min. The final PVA concentration was equal to that of the copolymers. The solution was evaporated overnight at room temperature to completely remove the organic phase and form a thin polymer film at the bottom. The film was then rehydrated with ultrapure Type I water and dispersed by gentle agitation utilizing a rotary plate shaker at room temperature, 200 rpm, for approximately 15 minutes. The dispersion was then sonicated in a bath sonicator at room temperature for 10 min or until it was translucent. The particle solution was prepared such that the final concentration of FGF2 was 100 μg/ml, or the final concentration of lysostaphin was 500 μg/ml.

**Example 22: Polymer-phosphate ink with LMW chitosan loaded with encapsulated FGF2**

Polymer-phosphate ink was prepared as in Example 17. To the prepared ink, encapsulated FGF2 protein was added in the amount to reach the final amount of 1 μg FGF2 per printed sample.

The FGF2 protein was encapsulated into core-shell particles based on a PLGA-PEG-PLGA copolymer prepared by double emulsion, water-in-oil-in-water method. The polymer concentration per formulation was 1.67 mg/ml in ultrapure Type I water. The nanoparticles used for incorporation into the paste had a diameter of 201.5 ± 25.9 nm and an encapsulation efficiency (EE) of 40.2%.

An adequate amount of alpha-tricalcium phosphate was added to the solid phase to keep the liquid-to-solid ratio of L/P = 0.62, thus compensating for the addition of the FGF2 protein solution.

**Example 23: Polymer-phosphate ink with LMW chitosan loaded with encapsulated lysostaphin**

Polymer-phosphate ink was prepared as in Example 17. To the prepared ink, encapsulated lysostaphin enzyme was added in the amount to reach the final amount of 10 μg lysostaphin per printed sample.

Lysostaphin was encapsulated into core-shell particles based on a PLGA-PEG-PLGA copolymer prepared by double emulsion, water-in-oil-in-water method. The polymer concentration per formulation was 1.67 mg/ml in ultrapure Type I water. The nanoparticles used for incorporation into the paste had a diameter of 177.3 ± 12.9 nm and an EE of 98.6%.

An adequate amount of alpha-tricalcium phosphate was added to the solid phase to keep the liquid-to-solid ratio of L/P = 0.62, thus compensating for the addition of the lysostaphin solution.

**Example 24: Polymer-phosphate ink printed specimens with LMW chitosan coated with FGF2**

Polymer-phosphate ink was prepared and specimens were printed as in Example 17.

The FGF2 was surface coated onto 10 specimens. The specimens were immersed in a solution of FGF2 protein having a concentration of 1 μg/ml and incubated for 1 hour at 25 °C. The coating efficiency (CE) of the protein on the surface of the samples was determined.

**Example 25: Polymer-phosphate ink printed specimens with LMW chitosan coated with lysostaphin**

Polymer-phosphate ink was prepared and specimens were printed as in Example 17.

The enzyme lysostaphin was surface coated onto 10 specimens. The specimens were immersed in a lysostaphin solution having a concentration of 10 μg/ml and incubated for 1 hour at 25 °C. The coating efficiency (CE) of the enzyme on the sample surface was determined.

In all examples, the release of FGF2 protein or the antibacterial enzyme lysostaphin, respectively, was monitored. The results are shown in Table 5. The release was monitored using an indirect enzyme-linked immunosorbent assay (ELISA). Samples from the protein calibration series and the elution solutions were applied to a 96-well microtiter plate and obtained by sampling at 3 h, 1, 2, 3, 7, 14, 21 and 28 days intervals in technical duplicates. The analysis was performed in the following steps: sample - blocking buffer - primary antibody - secondary antibody - TMB substrate - stop solution. Between steps, the wells were always washed with washing buffer PBST (phosphate buffered saline PBS 137 mM NaCl, 2.7 mM KCl, 8 mM $Na_2HPO_4$, 2 mM $KH_2PO_4$, pH 7.2 and 0.05 vol.% Tween-20). A 1% (w/v) solution of bovine albumin in PBST was used as a blocking buffer for FGF2 detection, and a 3% (w/v) solution of casein in PBST was used for lysostaphin detection. After the addition of TMB substrate (TMB-complete 2, TestLine Clinical Diagnostics s.r.o., Czech Republic), the reaction was terminated with 1M $H_2SO_4$ solution. The absorbance was measured on the samples using a plate-reader (BioTek, Agilent Technologies, USA) at a wavelength of 450 nm.

For FGF2 detection, a combination of primary antigen, monoclonal mouse FGF2 antibody (G-2) (sc-365106, Santa Cruz Biotechnology, USA) and secondary antigen, goat anti-mouse IgG-peroxidase Ab (A5278, Sigma, USA) was used. For the detection of lysostaphin, a combination of primary antigen, mouse IgG2b monoclonal antibody (MA1-21315, 6×-His Tag Monoclonal Antibody, Thermo Fisher Scientific, Czech Republic) and secondary antigen, goat anti-mouse IgG (H+L) peroxidase (62-6520, Thermo Fisher Scientific, Czech Republic) was used. The concentration of the samples was calculated using linear calibration curves, which were the result of the absorbance measurement of calibration series samples (determined for each microtiter plate separately). For FGF2, the calibration series ranged from 40-320 ng/ml protein in buffer (150 mM NaCl, 20 mM phosphate buffer, pH 7.5); for lysostaphin, the calibration series ranged from 66.2-2500 ng/ml protein in buffer (150 mM NaCl, 20 mM phosphate buffer, pH 7.5).

Table 5: The amounts of released FGF2 and Lysostaphin

| Time (days) | Cumulative release (%) | | | | | |
|---|---|---|---|---|---|---|
| | Example 19 | Example 20 | Example 22 | Example 23 | Example 24 | Example 25 |
| | FGF2 | Lysostaphin | FGF2 | Lysostaphin | FGF2 | Lysostaphin |
| | loaded into the paste | loaded into the paste | loaded into the paste | loaded into the paste | surface-coated | surface-coated |
| | plain | plain | encapsulated | encapsulated | plain | plain |
| EE (%) | | | 40.18 ± 0.22 | 98.65 ± 7.18 | | |
| CE (%) | | | | | 90.53 ± 2.37 | 80.65 ± 9.21 |
| 0.125 | 0.56 ± 0.08 | 4.54 ± 1.99 | 0.75 ± 0.29 | 5.40 ± 2.81 | 1.87 ± 0.96 | 12.32 ± 4.60 |
| 1 | 0.79 ± 0.15 | 12.00 ± 2.37 | 1.04 ± 0.22 | 11.48 ± 4.02 | 3.02 ± 1.08 | 20.96 ± 6.58 |
| 2 | 0.95 ± 0.09 | 16.27 ± 2.53 | 3.14 ± 2.28 | 16.00 ± 4.39 | 5.92 ± 5.81 | 29.07 ± 5.89 |
| 3 | 1.17 ± 0.03 | 19.68 ± 2.58 | 9.64 ± 5.38 | 23.21 ± 8.32 | 6.10 ± 5.70 | 35.92 ± 5.27 |
| 7 | 1.41 ± 0.20 | 26.63 ± 3.29 | 13.87 ± 1.90 | 30.39 ± 13.94 | 15.45 ± 2.33 | 45.06 ± 6.58 |
| 14 | 1.70 ± 0.32 | 31.16 ± 4.64 | 14.49 ± 1.93 | 37.20 ± 15.69 | 20.00 ± 3.70 | 53.02 ± 6.88 |
| 21 | 2.16 ± 0.28 | 33.42 ± 4.52 | 15.35 ± 2.62 | 41.52 ± 13.55 | 20.20 ± 3.76 | 61.61 ± 5.42 |
| 28 | 2.37 ± 0.16 | 35.17 ± 5.50 | 15.46 ± 2.62 | 43.94 ± 12.02 | 21.76 ± 4.31 | 67.08 ± 2.81 |

For **Example 19** and **Example 22,** the effect of FGF2 protein encapsulation on FGF2 release from the specimens was investigated. A significantly higher amount of released protein was observed for Example 22 compared to Example 19 at the monitored time intervals. A more significant difference in the amount of released FGF2 occurred after 2 days of monitoring, as shown in Table 5. The total amount measured after 28 days was 2.37 ± 0.16% for Example 19 and 15.46 ± 2.62% for Example 22. This difference was due to FGF2 being additionally protected by the PLGA-PEG-PLGA capsule in the form of "core-shell" particles in Example 22. Due to the needle-like structure (CDHA phase) of the cured cement, the release of FGF2 from the implant was not blocked in Example 22 compared to Example 19, where a very low amount of protein was released due to mechanical hindrance.

For **Example 20** and **Example 23,** the effect of encapsulation of the enzyme lysostaphin on lysostaphin release from the specimens was investigated. In Example 23, it was found that the total amount of released protein was significantly higher at the monitored time intervals compared to Example 20. According to the data in Table 5, a significant difference between Example 23 and Example 20 was evident after 2 days already, where the enzyme was released according to the first- order kinetics. In Example 23, only a portion of the total enzyme dose was released by day 2, followed by a second release phase that continued until day 28, with 43.94 ± 12.02% of the total encapsulated enzyme being released. In contrast, in Example 20, 35.17 ± 5.50% of the total encapsulated enzyme was released after 28 days. Example 23 effectively demonstrated the benefit of PLGA-PEG-PLGA based "core-shell" particles, which not only protected the enzyme from external conditions but also enhanced its gradual release from cured cement.

For **Example 19** and **Example 24,** the difference between the incorporation of FGF2 protein into the sample and its FGF2 deposition (coating) onto the sample surface was investigated. The efficiency of protein coating was determined to be 90.53 ± 2.37%. A significantly higher amount of released protein was observed for Example 24 at the monitored time intervals compared to Example 19. A noticeable difference in the amount of released FGF2 occurred as early as 3 hours of monitoring, as shown in Table 5, where there was a significant increase in released protein for Example 24 as opposed to Example 19. FGF2 was released by a first-order mechanism for Example 24 with a slight stagnation between days 2 and 3 of monitoring. The total amount of FGF2 released after 28 days was 2.37 ± 0.16% for Example 19 and 21.76 ± 4.31% for Example 24. In Example 24, the protein was not mixed into the ink prior to printing but was applied to the surface of the finished implant specimens. Thus, in Example 24, the release of the protein was not mechanically inhibited but was retarded by weak binding interactions of the protein with groups present on the surface of the implant material.

In **Example 20** and **Example 25,** the direct incorporation of the lysostaphin enzyme into the sample was compared to its coating onto the specimen. The efficiency of applying the enzyme onto the sample was $80.65 \pm 9.21\%$ of the total applied enzyme concentration. In Example 25, where the enzyme was coated onto the surface of the specimen, a significantly higher amount of enzyme was released after 28 days, $67.08 \pm 2.81\%$, whereas in Example 20, where the enzyme was directly incorporated into the sample, only $35.17 \pm 5.50\%$ of the total amount of enzyme was released. The release of the enzyme in Example 20 followed first-order kinetics, while in Example 25 a constant release according to zero-order kinetics was observed during the first 3 days, followed by a gradual release according to first-order kinetics after the 3$^{rd}$ day. The samples of Example 25 showed a significantly higher enzyme release after 3 hours of the study, with $12.32 \pm 4.60\%$ of the total enzyme released, compared to the samples of Example 20, where only $4.54 \pm 1.99\%$ of the enzyme was released after this time. There is a noticeable difference between the samples due to the different methods of enzyme application. In Example 25, the enzyme was not incorporated directly into the ink before printing, which prevented weak binding interactions between the enzyme and groups present in the implant material, thereby achieving a more efficient release of enzyme from the sample compared to Example 20, where the enzyme was incorporated directly into the material and enzyme release was inhibited.

### Example 26: Polymer-phosphate ink printed specimens with LMW chitosan coated with both FGF2 and lysostaphin

Polymer-phosphate ink was prepared and specimens were printed as in Example 17.

The FGF2 and lysostaphin were surface coated onto 10 specimens. The specimens were immersed in a solution of FGF2 protein having a concentration of 1 $\mu$g/ml and lysostaphin solution having a concentration of 10 $\mu$g/ml. The specimens were incubated for 1 hour at 25 °C. The coating efficiency (CE) of the protein on the surface of the samples was determined.

### Example 27: In vitro cytotoxicity testing

During the in vitro tests, the cytotoxicity of the materials from Example 17, Example 18, Example 24, Example 25 and Example 26 was verified.

The human osteoblast line SAOS-2 was used as a cell model and the cytotoxicity of the materials was tested by two methods - measuring cell viability and staining cells with viable dye followed by photodocumentation.

All work was carried out under sterile conditions. The human osteoblast cell line SAOS-2 (DSMZ, Germany) was cultured in McCoy's 5A phenol red-free medium (Merck KGaA, Germany) containing 10% v/v fetal bovine serum (Thermo Fisher Scientific, USA), 100 U/l penicillin, 0.1 mg/ml streptomycin (Biosera, France) and 1% v/v streptomycin (Biosera, France). L-Glutamine (Thermo Fisher Scientific). Cells were cultured at 37°C and 5% CO2 to 80% confluence and then used for the experiment. The cell passages used ranged from 4-7 passages. Sterile printed samples were loaded into 48-well plates (Techno Plastic Products, Switzerland) and cells were seeded onto the materials at a rate of 200,000 cells/sample in a 20 $\mu$l droplet volume and applied directly to the 3D structure of the sample. After 5 minutes, 300 $\mu$l of culture medium was carefully added to the samples and the cells were cultured for 4 hours at 37°C and 5% $CO_2$.

To measure metabolic activity, samples with cells were loaded into new 48-well plates (Techno Plastic Products), and 500 $\mu$l of alamarBlue solution (Thermo Fisher Scientific) diluted $10\times$ in culture medium was added. Samples were incubated for 2 h at 37°C and 5% CO2 and then $4\times 100$ $\mu$l was pipetted into black 96-well panels (Thermo Fisher Scientific) and fluorescence was measured at 530 nm (ex) and 590 nm (em) in a plate analyzer (Synergy HT, Biotek, USA). Each sample was analyzed in 5-8 replicates. To visualize live cells deposited on the samples, the samples were transferred to new 48-well plates (Techno Plastic Products) and rinsed $1\times$ with PBS. Viable calcein-AM dye (Merck) was diluted $1000\times$ in culture medium without phenol red and without fetal bovine serum, and 300 $\mu$l was applied to the samples. These were subsequently incubated for 30 min at 37°C and 5% CO2, rinsed with medium and imaged microscopically using an Olympus IX-83 microscope, UPlanFL N $10\times$/0.30 objective, Olympus UPlanFL N $20\times$/0.50 objective and FITC laser. Twenty images covering the entire sample area were taken from each sample, and these were then compressed into a single image of the entire sample using VisiView software (Olympus).

Measurement of metabolic activity showed that there was no difference in this monitored parameter between the samples from Example 24, Example 25 and Example 26 compared to the samples from Example 17 (tested by unpaired t-test, p=0.05). Cells adhered to the 3D printed materials processed viable dye showed a fluorescent signal, and thus their viability was demonstrated visually.

### Example 28: In vivo tests of osseointegration using a laboratory rat model

**Table 6.** List of printed implants applied in the final animal study. 4 types of implants and 2 time points (TP) were included. The numbers represent the number of animals.

| Type of implant | Implant description | TP1 = 3 weeks | TP2 = 3 months |
|---|---|---|---|
| EMPTY | defect without any treatment, spontaneous healing | 12 | 12 |
| Ex. 17 - 1.8 LMW CHIT | bone paste with chitosan (low MW, 1.8%) | 12 | 12 |
| Ex. 18 - 1.8 HMW CHIT | bone paste with chitosan (high MW, 1.8%) | 12 | 12 |
| Ex. 26 - 1.8 LMW CHIT + HIGH COAT | bone paste with chitosan (low MW, 1.8%) and with FGF2 + LYSS (1 $\mu$g/cm$^2$ + 10 $\mu$g/cm$^2$) | 12 | 12 |

In the implants prepared according to Example 17, Example 18, and Example 26 the osseointegration of cranial bone implants was verified on the parietal bone of a laboratory rat and both the safety of the implants and the promotion of new tissue formation in the defect were demonstrated. The size of the implants was adjusted to the largest possible defect size in the rat skull and was 3.5 $\times$ 1.0 mm (diameter $\times$ height). The model chosen for the osseointegration test was a laboratory rat model, Wistar strain, male, at least 6 months old, because of the sufficient skeletal size. 48 animals were included in the experiment, and each animal received 2 implants according to the predetermined randomization of the experiment. This allowed a total of 96 samples to be tested - 3 types of materials + control for spontaneous healing at 2-time points and at N = 12 per experimental group. After induction of general anesthesia, rats were immobilized in a precise stereotactic construct to avoid changing the head position. Under sterile conditions, rats were subjected to 2 defects of 3.5 mm diameter each in the cranial parietal bone. The defects were filled with prepared 3D printed implants. The defects included controls of spontaneous healing, i.e. the group of defects was left without filling. Subsequently, the wounds were sutured and treated with Novikov solution. Within 15 min of recovery from anesthesia, the animals regained full consciousness and subsequently received food and water ad libitum, i.e. the surgery did not have a significant negative effect on their health status. All wounds remained intact until the end of the experiment. After 3 weeks and 3 months, the experiments were terminated, and the cranial bones with defects at certain stages of healing were explanted, photographically documented and immediately placed in 4% formaldehyde for fixation and preservation for subsequent analyses. All samples were analyzed first by micro-CT and then by qualitative histology. For each material type and time point, 12 replicates were performed, i.e. N = 12 for each experimental group.

Micro-CT evaluation (for Example 26 - combination of LYS and FGF):

All samples were scanned in 4% formaldehyde solution. The scans were performed in a Phoenix Nanotom S x-ray micro-CT system from Waygate Technologies / Baker Hughes Digital Solutions GmbH. An acceleration voltage of 90 kV was used and a current setting of 90 $\mu$A. The voxel size was 11.0 $\mu$m and the geometrical magnification was 4.55. 1500 images were collected with an exposure time of 1.5 s and averaging of 3 images. No x-ray filter was used. The method allows visualization of bone and calcified tissue and qualitative and quantitative evaluation of bone formation and implant degradation. Qualitative analysis of samples after 3 months showed partial integration of the implants in form of direct bonding to the surrounding bone in case of implants prepared according to Examples 17 and 18. Implants prepared according to Example 26 showed sporadic bonding. Quantification was performed by calculating the total volume of original bone, implant and newly formed calcified tissue within a virtual cylinder of fixed size, positioned around the implant, or around an empty defect in the case of controls. After 3 weeks, no statistically significant difference in terms of total volume could be observed between the groups. After 3 months, implants prepared according to Examples 17 and 18 performed better with a statistically significant difference compared to implants prepared according to Example 26.

Histological evaluation:

All types of experimental samples were first subjected to micro-CT evaluation and then to qualitative histological analysis. After explantation from the terminated animals, the samples were fixed in 4% formaldehyde for 1 month. Subsequently, they were decalcified with Osteosens solution (Biognost, Croatia) for 5-8 weeks; the solution was changed once a week. The sufficient level of decalcification was verified by the needle puncture method. Then, the bone sample was trimmed with a scalpel so that approximately 1 mm of original bone tissue was still around the healing defects with implants. On visual inspection of the healing defects, all implants were clearly visible and there was no loss. Subsequently, the bone samples were dehydrated with a growing ethanol series (70-99%), transferred to xylene and paraffin, using tissue processor TP1020 (Leica, USA). This was followed by embedding the samples in paraffin blocks using an embedding station Arcadia (Leica, USA).Next, the samples were cut into 5-$\mu$m sections using a microtome (Leica, USA), stretched on a slide and stained with basic histological hematoxylin-eosin stain. During each step of the histological procedure, attention was paid on the orientation of samples and sections to keep the right and left positions of implants in parietal bone. For qualitative histological analysis we are able to distinguish implant matrix, new bone, old bone, connective tissue, immune infiltration. The stained slides were scanned using a SLIDEVIEW VS200 scanner with OlyVIA software (Olympus, Japan).

Qualitative histological analysis showed that all types of specimens were interspersed with new connective tissue and in a number of specimens this tissue replicated the filament structure of the implants, suggesting the suitability of the 3D implant structure for promoting the healing process of bone defects in the skull. Depending on the stage of healing, areas of newly formed tissue could be observed directly in the porous filament structure of the implants. These islets were often nourished by the newly formed capillaries and the tissue was everywhere in close contact with the porous structure of the implants without signs of massive immune infiltration. In most specimens, new bone formation was observed directly from the newly formed connective tissue. The new bone tissue was formed by osteoblasts arising directly from the connective tissue. This suggests a so-called desmodenous mechanism of bone formation (i.e., straight bone is formed from connective tissue without a cartilage phase), a process typical for flat bones such as the skull. Thus, the 3D printed implants promote the regeneration of cranial bone defects.

Industrial Applicability

The invention is useful for the preparation of tissue implants, e.g. bone implants which promote the healing of bone defects and facilitate the formation of bone tissue.

**Claims**

1. A kit for preparation of composition for low-temperature 3D printing of tissue implants, wherein the kit contains:

   - calcium phosphate powder,
   - chitosan particles,
   - aqueous solution of block copolymer (D,L-polylactide-*co*-polyglycolide)-*b*-poly(ethyleneglycol)-*b*-(D,L-polylactide-*co*-polyglycolide) (PLGA-PEG-PLGA).

2. Composition for low-temperature 3D printing of tissue implants, said composition containing

   - calcium phosphate powder,
   - chitosan particles,
   - aqueous solution of block copolymer (D,L-polylactide-*co*-polyglycolide)-*b*-poly(ethyleneglycol)-*b*-(D,L-polylactide-*co*-polyglycolide) (PLGA-PEG-PLGA).

3. Composition according to claim 2, wherein the aqueous solution of the block copolymer PLGA-PEG-PLGA has a concentration within the range of 3 to 30 wt. %, and
   wherein the weight ratio of the aqueous solution of the block copolymer PLGA-PEG-PLGA to the sum of calcium phosphate powder and chitosan particles is within the range 0.3 to 0.65.

4. The kit according to claim 1 or the composition according to claim 2 or 3, wherein the amount of chitosan particles is 0.1 to 5 wt. %, preferably 0.3 to 3 wt.%, relative to the sum of weights of calcium phosphate powder and chitosan particles.

5. The kit according to claim 1 or 4 or the composition according to any one of claims 2 to 4, wherein the (D,L-polylactide-*co*-polyglycolide)-*b*-poly(ethyleneglycol)-*b*-(D,L-polylactide-*co*-polyglycolide) block copolymer preferably has the general formula $(PLA_x/PGA_y)-PEG_z-(PLA_x/PGA_y)$, wherein the degree of polymerization z for PEG is in the range from 22 to 35, the degree of polymerization 2x for LA is in the range from 16 to 41 and the degree of polymerization 2y for GA is in the range from 6 to 18.

6. The kit according to any one of claims 1, 4, 5 or the composition according to any one of claims 2 to 5, wherein the aqueous solution of the block copolymer PLGA-PEG-PLGA further comprises at least one plasticizer which is polyethylene glycol with a molecular weight of 200 to 1000 g/mol, and/or at least one polysorbate-based emulsifier, wherein the total amount of plasticizer(s) and/or polysorbate-based emulsifier(s) preferably corresponds to a concentration of 2 to 6 wt. %, relative to the weight of the aqueous solution of PLGA-PEG-PLGA.

7. The kit according to any one of claims 1, 4 to 6, or the composition according to any one of claims 2 to 6, wherein the calcium phosphate powder has a particle size in the range of 1 to 200 micrometers, more preferably in the range of 1 to 100 micrometers, and/or the calcium phosphate powder has a median particle size d50 of 1 to 50 micrometers, more preferably 1 to 20 micrometers.

8. The kit according to any one of claims 1, 4 to 7 or the composition according to any one of claims 2 to 7, wherein chitosan has a weight-average molecular weight within the range from 2 to 400 kg/mol, and/or a degree of deacetylation from 35 to 70%, and/or an average particle size of less than 20 micrometers.

9. The kit according to any one of claims 1, 4 to 8 or the composition according to any one of claims 2 to 8, which further comprises at least one antimicrobial enzyme and/or at least one growth factor, which are preferably selected from proteolytic enzymes, polysaccharide degrading enzymes, fibroblast growth factors FGF1 to FGF10 and FGF16 to FGF23, epidermal growth factors, vascular endothelial growth factors, and bone morphogenetic proteins from the range of BMP-1 to BMP-8, BMP-10, BMP-11 and BMP-15.

10. The kit or the composition according to claim 8, wherein the antimicrobial enzyme(s) and/or growth factor(s) is/are in the form of core-shell particles in which the antimicrobial enzyme(s) and/or growth factor(s) is/are encapsulated in a lipidic or polymeric carrier, preferably in a polymeric carrier which is PLGA-PEG-PLGA polymersome.

11. A method of preparing a composition for low-temperature 3D printing according to any one of claims 2 to 10, said method comprising the steps of:

   - providing an aqueous solution of block copolymer (D,L-polylactide-*co*-polyglycolide)-*b*-poly(ethyleneglycol)-*b*-(D,L-polylactide-*co*-polyglycolide) (PLGA-PEG-PLGA) ;
   - dissolving chitosan in the aqueous solution of the block copolymer PLGA-PEG-PLGA;
   - providing calcium phosphate powder;
   - combining the aqueous solution of the block copolymer PLGA-PEG-PLGA and chitosan with calcium phosphate powder.

12. A method of low-temperature 3D printing of tissue implants using the kit according to any one of claims 1, 4-10, said method comprising the steps of:

   - providing an aqueous solution of block copolymer (D,L-polylactide-*co*-polyglycolide)-*b*-poly(ethyleneglycol)-*b*-(D,L-polylactide-*co*-polyglycolide) (PLGA-PEG-PLGA);
   - dissolving chitosan in the aqueous solution of the block copolymer PLGA-PEG-PLGA;
   - providing calcium phosphate powder;
   - combining the aqueous solution of the block copolymer PLGA-PEG-PLGA and chitosan with calcium phosphate powder to produce a printable composition;
   - printing tissue implant(s) from the printable composition at the temperature within the range from 0 to 45 °C, preferably 10 to 37 °C, preferably using discontinuous extrusion method;
   - curing the printed object(s) in aqueous liquid or in air with at least 95 % of humidity.

13. A tissue implant comprising PLGA-PEG-PLGA, chitosan, calcium phosphate, calcium-deficient hydroxyapatite, and optionally at least one antimicrobial enzyme and/or at least one growth factor.

14. The tissue implant according to claim 11 or 12, which is surface-coated by at least one antimicrobial enzyme and/or at least one growth factor, which are preferably selected from proteolytic enzymes, polysaccharide degrading enzymes, fibroblast growth factors FGF1 to FGF10 and FGF16 to FGF23, epidermal growth factors, vascular endothelial growth factors, and bone morphogenetic proteins from the range of BMP-1 to BMP-8, BMP-10, BMP-11 and BMP-15.

| | Europäisches Patentamt European Patent Office Office européen des brevets | **EUROPEAN SEARCH REPORT** | **Application Number** EP 24 17 7422 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HAN SHUANG ET AL: "Programmed release of vascular endothelial growth factor and exosome from injectable chitosan nanofibrous microsphere-based PLGA-PEG-PLGA hydrogel for enhanced bone regeneration", INTERNATIONAL JOURNAL OF BIOLOGICAL MACROMOLECULES, vol. 253, 1 December 2023 (2023-12-01), page 126721, XP093218028, NL ISSN: 0141-8130, DOI: 10.1016/j.ijbiomac.2023.126721 * abstract * * pages 2-3, paragraph 2.4-2.5 * * Scheme 1 * * page 11, column 1 * | 1-14 | INV. A61L27/46 A61L27/54 B33Y10/00 B33Y80/00 |
| A | CHAMRADOVÁ I. ET AL: "The effect of hydroxyapatite particle size on viscoelastic properties and calcium release from a thermosensitive triblock copolymer", COLLOID & POLYMER SCIENCE, [Online] vol. 295, no. 1, 26 November 2016 (2016-11-26), pages 107-115, XP093218110, DE ISSN: 0303-402X, DOI: 10.1007/s00396-016-3983-7 Retrieved from the Internet: URL:http://link.springer.com/article/10.1007/s00396-016-3983-7/fulltext.html> [retrieved on 2024-10-24] * abstract * * paragraph "Materials and methods"; pages 108-109 * | 1-14 | TECHNICAL FIELDS SEARCHED (IPC) B33Y A61L |

-/--

The present search report has been drawn up for all claims

| Place of search | Date of completion of the search | Examiner |
|---|---|---|
| The Hague | 25 October 2024 | Guazzelli, Giuditta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 24 17 7422

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VOJTOVA LUCY ET AL: "The Effect of the Thermosensitive Biodegradable PLGA-PEG-PLGA Copolymer on the Rheological, Structural and Mechanical Properties of Thixotropic Self-Hardening Tricalcium Phosphate Cement", INTERNATIONAL JOURNAL OF MOLECULAR SCIENCES, vol. 20, no. 2, 17 January 2019 (2019-01-17), page 391, XP093218111, Basel, CH ISSN: 1422-0067, DOI: 10.3390/ijms20020391 * abstract * * pages 3-4, paragraphs 2.2.2, 2.2, 2.3 * ----- | 1-14 | |
| | | | TECHNICAL FIELDS SEARCHED (IPC) |

The present search report has been drawn up for all claims

| Place of search | Date of completion of the search | Examiner |
|---|---|---|
| The Hague | 25 October 2024 | Guazzelli, Giuditta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DUARTE, M.L** ; **FERREIRA, M.C** ; **MARVAO, M.R** ; **ROCHA, J**. An optimised method to determine the degree of acetylation of chitin and chitosan by FTIR spectroscopy. *International Journal of Biological Macromolecules*, 2002, vol. 31 (1-3), 1-8
- **ALTIOK, D.** ; **ALTIOK, E.** ; **TIHMINLIOGLU, F.** Physical, antibacterial and antioxidant properties of chitosan films incorporated with thyme oil for potential wound healing applications. *Journal of Materials Science: Materials in Medicine*, 2010, vol. 21 (7), 2227-2236

- **HIRAI, A.** ; **ODANI, H.** ; **NAKAJIMA, A.** Determination of degree of deacetylation of chitosan by H NMR spectroscopy. *Polymer Bulletin*, 1991, vol. 26 (1), 87-94
- **LAVERTU, M.** ; **XIA, Z.** ; **SERREQI, A.N.** ; **BERRADA, M.** ; **RODRIGUES, A.** ; **WANG, D.** ; **BUSCHMANN, M.D.** ; **GUPTA, A.** A validated 1H NMR method for the determination of the degree of deacetylation of chitosan. *Journal of Pharmaceutical and Biomedical Analysis*, 2003, vol. 32 (6), 1149-1158